(19) Europäisches Patentamt
European Patent Office
Office européen des brevets

(11) **EP 3 898 985 B1**

(12) **EUROPEAN PATENT SPECIFICATION**

(45) Date of publication and mention
of the grant of the patent:
**08.02.2023 Bulletin 2023/06**

(21) Application number: **19817783.4**

(22) Date of filing: **17.12.2019**

(51) International Patent Classification (IPC):
*C12N 15/80* (1990.01)   *C12N 15/62* (1990.01)

(52) Cooperative Patent Classification (CPC):
**C12N 15/80; C12N 9/20; C12N 15/625;**
C07K 2319/02; C07K 2319/50

(86) International application number:
**PCT/EP2019/085553**

(87) International publication number:
**WO 2020/127198 (25.06.2020 Gazette 2020/26)**

(54) **TANDEM PROTEIN EXPRESSION**

TANDEMPROTEINEXPRESSION

EXPRESSION DE PROTÉINES EN TANDEM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2018 EP 18215558**

(43) Date of publication of application:
**27.10.2021 Bulletin 2021/43**

(73) Proprietor: **Novozymes A/S
2880 Bagsvaerd (DK)**

(72) Inventors:
• **BAERENDS, Richard, Jan, Steven
2880 Bagsvaerd (DK)**
• **OLSEN, Carsten, Lillelund
2880 Bagsvaerd (DK)**
• **ARNAU, Josè
2880 Bagsvaerd (DK)**

(74) Representative: **NZ EPO Representatives
Krogshoejvej 36
2880 Bagsvaerd (DK)**

(56) References cited:
WO-A1-96/13580        WO-A1-2013/076253
WO-A1-2018/150021     WO-A2-2005/093050
CN-A- 104 962 573     US-A1- 2006 003 414

• SHEN H ET AL: "CELLOBIOHYDROLASE B, A
SECOND EXO-CELLOBIOHYDROLASE FROM
THE CELLULOLYTIC BACTERIUM
CELLULOMONAS FIMI", BIOCHEMICAL
JOURNAL, PUBLISHED BY PORTLAND PRESS
ON BEHALF OF THE BIOCHEMICAL SOCIETY,
vol. 1, no. 311, 1 October 1995 (1995-10-01), pages
67-74, XP001064588, ISSN: 0264-6021

Remarks:
The complete document including Reference
Table(s) and the Sequence Listing(s) can be
downloaded from the EPO website

**Description**

**Reference to sequence listing**

This application contains a Sequence Listing in computer readable form.

**FIELD OF THE INVENTION**

The present invention provides means and methods for improving polypeptide expression yield by increasing the number of polypeptides that can be transported into the endoplasmic reticulum (ER) per translocation event.

**BACKGROUND OF THE INVENTION**

Product development in industrial biotechnology includes a continuous challenge to increase enzyme yields at large scale to reduce costs. Two major approaches have been used for this purpose in the last decades. The first one is based on classical mutagenesis and screening. Here, the specific genetic modification is not pre-defined and the main requirement is a screening assay that is sensitive to detect relatively discrete increments in yield. High throughput screening enables large numbers of mutants to be screened in search for the desired phenotype, i.e., higher enzyme yields. The second approach includes numerous strategies ranging from the use of stronger promoters and multicopy strains to ensure high expression of the gene of interest to the use of codon optimized gene sequences to aid translation. However, high level production of a protein may trigger several bottlenecks in the cellular machinery for secretion of the enzyme of interest into the medium.

For secreted enzymes whose amino acid sequence includes a signal peptide (SP), translation is followed by cleavage of the SP by a signal peptidase and translocation of the maturing protein into the endoplasmic reticulum (ER; Voss et al., 2013; Aviram and Schuldiner 2017). To secrete a protein through the ER, the signal recognition particle (SRP) recognizes the SP in a highly conserved manner. The SRP associates with the ribosome and through a hydrophobic cleft recognizes secretory proteins with hydrophobic motifs as they are being translated and binds to the SRP receptor (SR) present in the ER membrane in eukaryotes (Aviram and Schuldiner 2017). Thus, a limiting step in the secretion of a protein might be at the translocation of the nascent polypeptide from the cytosol into the ER. Since this process is very energy demanding, there is an upper capacity limit as to the number of translocations per time unit per cell.

Means and methods for overcoming the upper translocation limit would constitute an entirely new approach to yield optimization and would thus be highly desirable within industrial biotechnology,

**SUMMARY OF THE INVENTION**

The present invention provides means and methods for improving polypeptide expression yield by increasing the number of polypeptides that can be transferred into the ER while still using one signal peptide per translocation-dependent event, thereby increasing the number of polypeptide units that can be secreted per translocation event.

In a first aspect, the present invention relates to a nucleic acid construct comprising a heterologous promoter operably linked to:

    a) a polynucleotide encoding a signal peptide; and
    b) at least two polynucleotides encoding one or more polypeptide of interest;

wherein the at least two polynucleotides encoding one or more polypeptide of interest are each separated by a linker polynucleotide encoding a linker polypeptide comprising a proteolytic cleavage site;
wherein the signal peptide, the one or more polypeptide of interest and the linker polypeptide(s) comprising a proteolytic cleavage site are encoded in frame as a single polypeptide; and wherein the at least two polynucleotides encode at least two polypeptides of interest consisting of the same amino acid sequence, and wherein the at least two polypeptides of interest are secreted as separate polypeptides consisting of the same amino acid sequence.

In a second aspect, the present invention relates to an expression vector comprising a nucleic acid construct of the first aspect.

In a third aspect, the present invention relates to a fungal host cell comprising a nucleic acid construct of the first aspect and/or an expression vector of the second aspect.

In a fourth aspect, the present invention relates to a method for producing one or more polypeptide of interest, the method comprising:

    a) providing a fungal host cell of the third aspect:

b) cultivating said host cell under conditions conducive for expression of the one or more polypeptide of interest; and optionally

c) recovering the one or more polypeptide of interest.

**BRIEF DESCRIPTION OF DRAWINGS**

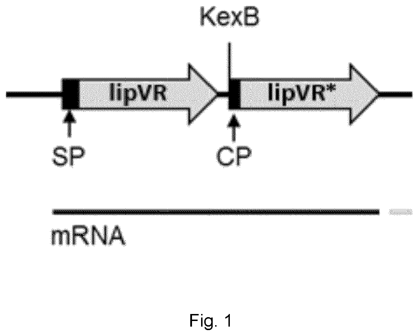

Fig. 1 shows schematically the lipVR expression cassette. Shortly, one or more "units" of the lipVR coding sequence (using different codon optimized version of the gene, lipVR, lipVR*, etc.) were cloned to make genetic constructions where lipVR is transcribed as a singlet, tandem or more. SP: native signal peptide from the lipVR gene or the cutinase prepro encoding sequence; CP: cutinase pro encoding sequence; KexB: KexB cleavage site ("KR"). In all cases, a single transcript contains all the "lipVR units".

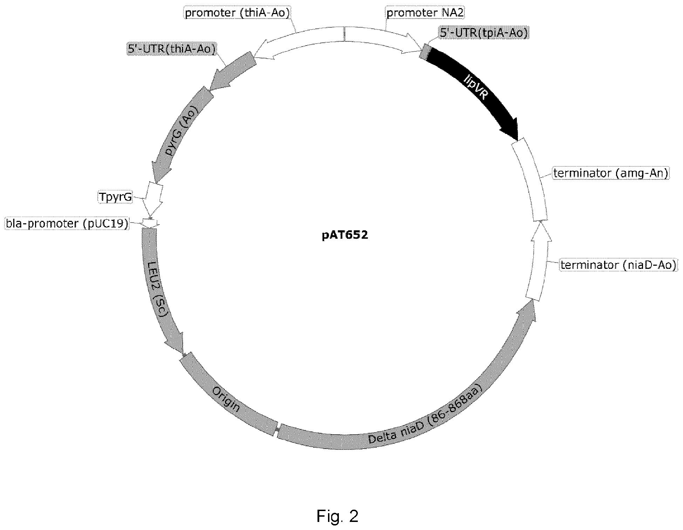

Fig. 2 shows a map of plasmid pAT652 for expression and secretion of the LipVR lipase in A. *oryzae* (single gene construct).

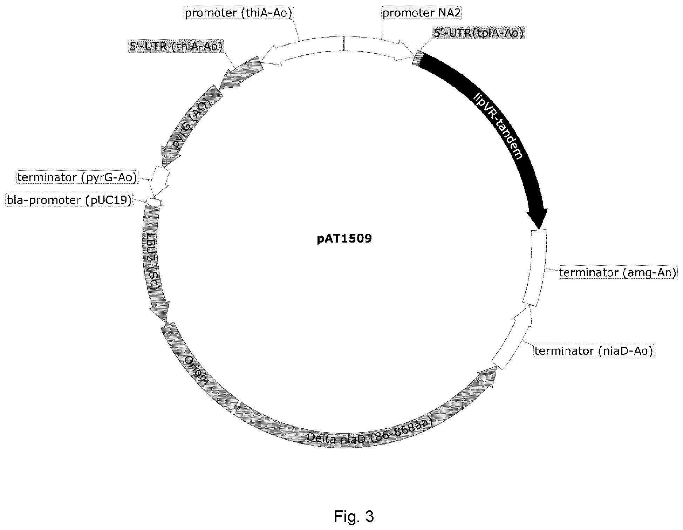

Fig. 3 shows a map of plasmid pAT1509 for expression and secretion of the LipVR lipase in *A. oryzae* (tandem gene construct).

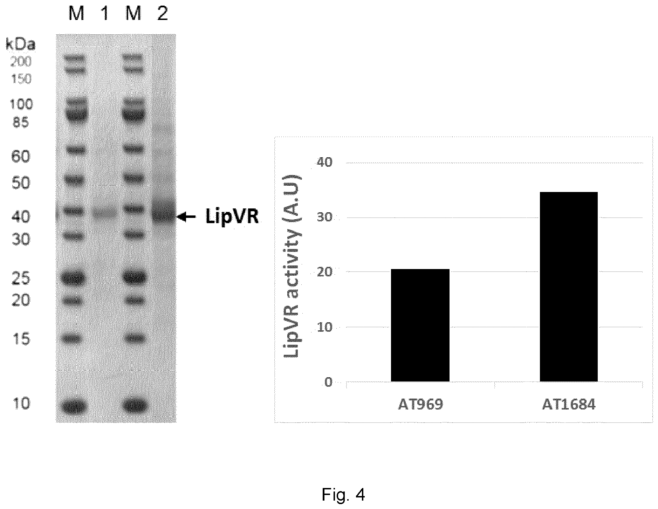

Fig. 4 (left) shows LipVR production as demonstrated by SDS PAGE for comparison of singlet vs. tandem production of LipVR by *A. oryzae* strains AT969 (lane 1: 18 copies of single expression plasmid pAT652) and AT1684 (lane 2: 9 copies of pAT1509 (lipVR tandem)) at the end of fermentation (day 7). Fig. 4 (right) shows lipolytic activity (depicted as arbitrary units, A.U.) at the end of fermentation of strain AT969 and AT1684.

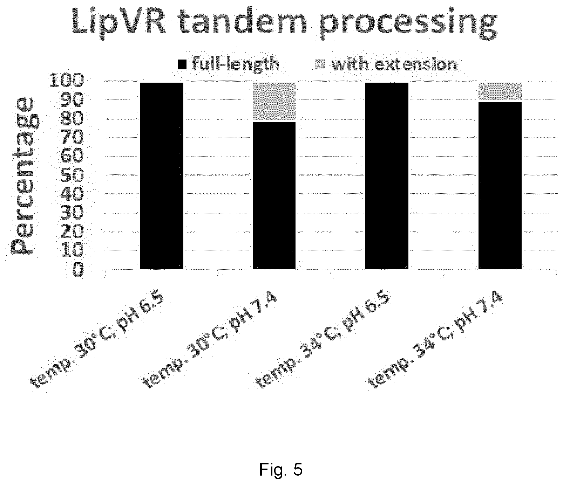

Fig. 5 shows the degree of correct processing to yield intact LipVR molecules produced during fed-batch fermentation of *A. oryzae* strain AT1684 containing the lipVR-tandem cassette. Samples (supernatant) were taken at the end of fermentation at 30 °C or 34 °C and at pH 6.5 or 7.4 as indicated and were analyzed by mass spectrometry. The level of tandem processing is shown as percentage of full length LipVR molecules (black boxes) and the aggregated percentages of differently but non-fully processed LipVR molecules (grey boxes).

**DEFINITIONS**

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor mRNA that is processed through a series of steps, including RNA splicing, before appearing as mature mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (i.e., from the same gene) or foreign (i.e., from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Heterologous promoter:** The term "heterologous promoter" means a promoter that is foreign (i.e., from a different gene) to the polynucleotide to which it is operably linked.

**Host cell:** The term "host cell" means any cell type that is susceptible to transformation, transfection, transduction, or the like with a nucleic acid construct or expression vector comprising a polynucleotide of the present invention.

The term "host cell" encompasses any progeny of a parent cell that is not identical to the parent cell due to mutations that occur during replication.

**Isolated:** The term "isolated" means a substance in a form or environment that does not occur in nature.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 1 to 289 of SEQ ID NO: 5 (corresponding to amino acids 1 to 289 of SEQ ID NO: 6). Amino acids -20 to -1 of SEQ ID NO: 5 are a signal peptide. It is known in the art that a host cell may produce a mixture of two of more different mature polypeptides (i.e., with a different C-terminal and/or N-terminal amino acid) expressed by the same polynucleotide. It is also known in the art that different host cells process polypeptides differently, and thus, one host cell expressing a polynucleotide may produce a different mature polypeptide (e.g., having a different C-terminal and/or N-terminal amino acid) as compared to another host cell expressing the same polynucleotide.

**Mature polypeptide coding sequence:** The term "mature polypeptide coding sequence" means a polynucleotide that encodes a mature polypeptide having lipolytic activity as determined in WO 2018/150021. In one aspect, the mature polypeptide coding sequence is nucleotides 61 to 1056 of SEQ ID NO: 3 or the cDNA sequence thereof (corresponding to nucleotides 61 to 927 of SEQ ID NO: 4) and nucleotides 1 to 60 of SEQ ID NO: 3 (corresponding to nucleotides 1 to 60 of SEQ ID NO: 4) encode a signal peptide.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Secreted:** The term "secreted" means that the one or more polypeptide of interest is processed and released into an extracellular environment, such as a growth medium. In one embodiment, the one or more polypeptide of interest is processed and released via the conventional secretion pathway comprising the endoplasmatic reticulum, Golgi apparatus, and secretory vesicles. In another embodiment, the one or more polypeptide of interest is processed and released via the unconventional secretion pathway (Rabouille, Trends in Cell Biology 2017, vol. 27, pp. 230-240; Kim et al., Journal of Cell Science 2018, vol. 131, jcs213686).

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$\text{(Identical Residues x 100)/(Length of Alignment} - \text{Total Number of Gaps in Alignment)}$$

For purposes of the present invention, the sequence identity between two deoxyribonucleotide sequences is determined using the Needleman-Wunsch algorithm (Needleman and Wunsch, 1970, *supra)* as implemented in the Needle program of the EMBOSS package (EMBOSS: The European Molecular Biology Open Software Suite, Rice et al., 2000, supra*),* preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$\text{(Identical Deoxyribonucleotides x 100)/(Length of Alignment} - \text{Total Number of Gaps in Alignment)}$$

**Variant:** The term "variant" means a polypeptide comprising an alteration, *i.e.,* a substitution, insertion, and/or deletion, at one or more (e.g., several) positions compared to the corresponding native polypeptide. A substitution means replacement of the amino acid occupying a position with a different amino acid; a deletion means removal of the amino acid occupying a position; and an insertion means adding an amino acid adjacent to and immediately following the amino acid occupying a position.

**DETAILED DESCRIPTION OF THE INVENTION**

The present invention provides means and methods for improving polypeptide expression yield by increasing the number of polypeptides that can be transported into the ER while still using only one SP per translocation event, thereby increasing the number of polypeptide units that can be secreted per translocation event.

The inventive concept is based on the design of nucleic acid constructs comprising a heterologous promoter operably linked to a polynucleotide encoding a signal peptide and at least two polynucleotides encoding one or more polypeptide of interest. Each of the polynucleotides encoding a polypeptide of interest are separated by a linker polynucleotide that encodes a linker polypeptide comprising a proteolytic cleavage site that is recognized by a suitable protease (Fig. 1). The signal peptide, the one or more polypeptide of interest, and the linker polypeptide(s) are encoded in frame to allow translation as a single polypeptide. The term "encoded in frame" refers to the mature mRNA that is obtained after RNA splicing and removal of any introns present in the precursor mRNA.

The nucleic acid constructs of the invention may be transformed into suitable host cells, and fermentation of such host cells under suitable conditions will allow transcription of the polynucleotides followed by ribosomal translation of the resulting mRNA. The resulting polypeptide containing one signal peptide and one or more polypeptide of interest each separated by a linker polypeptide will subsequently be targeted into the secretory pathway as a single unit in one translocation event. Following proteolytic processing by a protease that recognizes the proteolytic cleavage site comprised by the linker polypeptide, the one or more polypeptide of interest is liberated and secreted into the extracellular medium.

As shown in the Examples disclosed herein, use of nucleic acid constructs of the invention in the construction of fungal host cells results in increased yield of the polypeptide of interest as well as uniform processing of the individual copies of the polypeptide of interest. The latter is especially important for functional polypeptides such as enzymes, since integrity of the primary structure is important for folding and correct formation of the tertiary structure, which is important for activity.

Thus, in a first aspect, the present invention relates to nucleic acid construct comprising a heterologous promoter operably linked to:

a) a polynucleotide encoding a signal peptide; and
b) at least two polynucleotides encoding one or more polypeptide of interest;

wherein the at least two polynucleotides encoding one or more polypeptide of interest are each separated by a linker polynucleotide encoding a linker polypeptide comprising a proteolytic cleavage site;

wherein the signal peptide, the one or more polypeptide of interest and the linker polypeptide(s) comprising a proteolytic cleavage site are encoded in frame as a single polypeptide, and
wherein the at least two polynucleotides encode at least two polypeptides of interest consisting of the same amino acid sequence, and wherein the at least two polypeptides of interest are secreted as separate polypeptides consisting of the same amino acid sequence.

Preferably, the nucleic acid constructs of the invention comprise at least two, e.g., 2, 3, 4, 5, 6, 7, 8, 9, 10, 11, 12, 13, 14, 15, 20, 25, 30, 35, 40, or more, polynucleotides encoding a one or more, e.g., 1, 2, 3, 4, 5, 6, 7, 8, 9, 10, 11, 12, 13, 14, 15, 20, 25, 30, 35, 40, or more, polypeptide of interest.

The signal peptide may be any signal peptide that is suitable for targeting the one or more polypeptide of interest to the secretory pathway of a host cell, preferably a fungal host cell. Preferably, the signal peptide is a heterologous signal peptide.

In a preferred embodiment, the signal peptide has a sequence identity of at least 80%, e.g., at least 85%, at least 90%, at least 91%, at least 92%, at least 93%, at least 94%, at least 95%, at least 96%, at least 97%, at least 98%, at least 99%, or 100%, to SEQ ID NO: 2 preferably the signal peptide comprises or consists of SEQ ID NO: 2.

In a preferred embodiment, the polynucleotide encoding the signal peptide has a sequence identity of at least 80%, e.g., at least 85%, at least 90%, at least 91%, at least 92%, at least 93%, at least 94%, at least 95%, at least 96%, at least 97%, at least 98%, at least 99%, or 100%, to SEQ ID NO: 1; preferably the polynucleotide encoding the signal peptide comprises or consists of SEQ ID NO: 1.

The one or more polypeptide of interest may be any polypeptide. Preferably, the polypeptide of interest comprises an enzyme; preferably the enzyme is selected from the group consisting of hydrolase, isomerase, ligase, lyase, oxidoreductase, or transferase; more preferably an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, and beta-xylosidase; most preferably the enzyme is a lipase.

In a preferred embodiment, the one or more polypeptide of interest has a sequence identity of at least 80%, e.g., at least 85%, at least 90%, at least 91%, at least 92%, at least 93%, at least 94%, at least 95%, at least 96%, at least 97%, at least 98%, at least 99%, or 100%, to the mature polypeptide of SEQ ID NO: 5; preferably the one or more polypeptide of interest comprises or consists of the mature polypeptide of SEQ ID NO: 5.

In a preferred embodiment, the at least two polynucleotides encoding one or more polypeptide of interest have a sequence identity of, independently, at least 80%, e.g., at least 85%, at least 90%, at least 91%, at least 92%, at least 93%, at least 94%, at least 95%, at least 96%, at least 97%, at least 98%, at least 99%, or 100%, to the mature polypeptide coding sequence of SEQ ID NO: 4; preferably the at least two polynucleotides encoding one or more polypeptide of interest comprise or consist of the mature polypeptide coding sequence of SEQ ID NO: 4; also preferably, the at least two polynucleotides encoding one or more polypeptide are different, i.e., not identical.

The nucleic acid constructs of the invention comprise at least two polynucleotides encoding at least two polypeptides of interest consisting of the same amino acid sequence, and the at least two polypeptides of interest are secreted as separate polypeptides having the same amino acid sequence. Preferably, the at least two polypeptides of interest have a sequence identity of at least 80%, e.g., at least 85%, at least 90%, at least 91%, at least 92%, at least 93%, at least 94%, at least 95%, at least 96%, at least 97%, at least 98%, at least 99%, or 100%, to the mature polypeptide of SEQ ID NO: 5; preferably the at least two polypeptides of interest comprise or consist of the mature polypeptide of SEQ ID NO: 5. Preferably, the at least two polynucleotides encoding the at least two polypeptides of interest have a sequence identity of, independently, at least 80%, e.g., at least 85%, at least 90%, at least 91%, at least 92%, at least 93%, at least 94%, at least 95%, at least 96%, at least 97%, at least 98%, at least 99%, or 100%, to the mature polypeptide coding sequence of SEQ ID NO: 4; preferably the at least two polynucleotides encoding the at least two polypeptides of interest comprise or consist of the mature polypeptide coding sequence of SEQ ID NO: 4; also preferably, the at least two polynucleotides encoding the at least two polypeptides are different, i.e., not identical.

The linker polynucleotide should be chosen so that the encoded linker polypeptide is of sufficient length and flexibility to allow the proteolytic cleavage site to be accessed by a suitable protease that recognizes the proteolytic cleavage site. Preferably, the linker polypeptide comprise at least 10 amino acids, e.g., at least 11 amino acids, at least 12 amino acids, at least 13 amino acids, at least 14 amino acids, at least 15 amino acids, at least 16 amino acids, at least 17 amino acids, at least 18 amino acids, at least 19 amino acids, at least 20 amino acids, at least 25 amino acids, at least 30 amino acids, at least 35 amino acids, at least 40 amino acids, at least 45 amino acids, at least 45 amino acids, at least 50 amino acids, at least 55 amino acids, at least 60 amino acids, at least 65 amino acids, at least 70 amino acids, at least 75 amino acids, at least 80 amino acids, at least 85 amino acids, at least 90 amino acids, at least 95 amino acids, at least 100 amino acids, or more.

The linker polypeptide comprises a proteolytic cleavage site that is recognized by a suitable protease, preferably a KexB protease. Preferably, the proteolytic cleavage site is a dibasic amino acid motif such as a Lys-Arg or an Arg-Arg motif. Preferably, the proteolytic cleavage site is a KexB cleavage site.

The one or more polypeptide of interest is expressed as part of a single polypeptide, but should be liberated intracellularly prior to secretion of the individual polypeptides of interest into the extracellular medium. Thus, preferably, the proteolytic cleavage site encoded by the linker polypeptide is recognized by an intracellular protease. Preferably, proteolytic cleavage takes place intracellularly inside a suitable host cell comprising a nucleic acid construct of the invention. Alternatively, proteolytic cleavage takes place extracellularly during and/or after cultivation of a suitable host cell comprising a nucleic acid construct of the invention.

**Nucleic Acid Constructs**

The present invention relates to nucleic acid constructs comprising polynucleotides encoding a signal peptide, one or more polypeptide of interest, and linker polypeptide(s) operably linked to one or more control sequences that direct the expression of the polynucleotides in a suitable host cell under conditions compatible with the control sequences.

The polynucleotides may be manipulated in a variety of ways to provide for their expression. Manipulation of a polynucleotide may be desirable or necessary depending on the nucleic acid construct, expression vector, and/or host cell into which the polynucleotide is being introduced. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including variant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase *(glaA)*, *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and variant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The selected promoter should be a heterologous promoter that is foreign (i.e., from a different gene) to the polynucleotide(s) to which it is operably linked.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be a leader, a non-translated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for As*pergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

In a preferred embodiment, the signal peptide has a sequence identity of at least 80%, e.g., at least 85%, at least 90%, at least 91%, at least 92%, at least 93%, at least 94%, at least 95%, at least 96%, at least 97%, at least 98%, at least 99%, or 100%, to SEQ ID NO: 2; preferably the signal peptide comprises or consists of SEQ ID NO: 2.

In a preferred embodiment, the signal peptide comprises or consists of SEQ ID NO: 2.

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound.

In yeast, the ADH2 system or GAL1 system may be used.

In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used.

Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

**Polynucleotides**

The present invention also relates to polynucleotides encoding a signal peptide, polypeptides encoding one or more polypeptide of interest, and linker polynucleotide(s) encoding linker polypeptide(s). In an embodiment, the polynucleotides have been isolated.

The techniques used to isolate or clone a polynucleotide are known in the art and include isolation from genomic DNA or cDNA, or a combination thereof. The cloning of the polynucleotides from genomic DNA can be effected, e.g., by using the well-known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of As*pergillus*, or a related organism and thus, for example, may be an allelic or species variant of the polypeptide encoding region of the polynucleotide.

Modification of a polynucleotide encoding a signal peptid, one or more polypeptide of interest, or linker polypeptide may be necessary for synthesizing polypeptides substantially similar to these polypeptides. The term "substantially similar" to the polypeptide refers to non-naturally occurring forms of the polypeptide.

**Expression Vectors**

In a second aspect, the present invention also relates to recombinant expression vectors comprising polynucleotides of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Suitable selectable markers for yeast host cells include, but are not limited to, ADE2, HIS3, LEU2, LYS2, MET3, TRP1, and URA3.

Suitable selectable markers for filamentous fungal host cell include, but are not limited to, *adeA* (phosphoribosylaminoimidazole-succinocarboxamide synthase), *adeB* (phosphoribosyl-aminoimidazole synthase), *amdS* (acetamidase), *argB* (ornithine carbamoyltransferase), bar (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome. For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra).*

## Host Cells

In a third aspect, the present invention also relates to recombinant host cells comprising a nucleic acid construct or expression vector of the invention. A construct or vector comprising polynucleotides of the invention is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of host cell will to a large extent depend upon the polynucleotides encoding the one or more polypeptide of interest and their source.

The host cell may be any cell useful in the recombinant production of a polypeptide of interest, e.g., a eukaryotic cell, preferably a fungal cell.

"Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast *(Endomycetales),* basidiosporogenous yeast, and yeast belonging to the *Fungi Imperfecti (Blastomycetes).* Since the classification of yeast may change in the future, for the purposes of this invention, yeast shall be defined as described in *Biology and Activities of Yeast* (Skinner, Passmore, and Davenport, editors, *Soc. App. Bacteriol. Symposium Series* No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

## Methods of Production

In a fourth aspect, the present invention also relates to methods of producing one or more polypeptide of interest, comprising:

a) providing a fungal host cell of the present invention;

b) cultivating the host cell under conditions conducive for production of the one or more polypeptide of interest; and optionally

c) recovering the one or more polypeptide of interest.

In one embodiment, the host cell is an *Aspergillus* cell. In a preferred embodiment, the host cell is an *Aspergillus oryzae* cell.

The fungal host cells are cultivated in a nutrient medium suitable for production of the polypeptide of interest using methods known in the art. For example, the cells may be cultivated by shake flask cultivation, or small-scale or large-scale fermentation (including continuous, batch, fed-batch, or solid-state fermentations) in laboratory or industrial fermentors in a suitable medium and under conditions allowing the polypeptide of interest to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the polypeptide of interest is secreted into the nutrient medium, the polypeptide of interest can be recovered directly from the medium. If the polypeptide of interest is not secreted, it can be recovered from cell lysates.

The one or more polypeptide of interest may be detected using methods known in the art that are specific for the polypeptide. These detection methods include, but are not limited to, use of specific antibodies, formation of an enzyme product, or disappearance of an enzyme substrate. For example, an enzyme assay may be used to determine the activity of the polypeptide of interest.

The one or more polypeptide of interest may be recovered using methods known in the art. For example, the one or more polypeptide of interest may be recovered from the nutrient medium by conventional procedures including, but not limited to, collection, centrifugation, filtration, extraction, spray-drying, evaporation, or precipitation. In one aspect, a fermentation broth comprising the one or more polypeptide of interest is recovered.

The one or more polypeptide of interest may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the one or more polypeptide of interest is not recovered, but rather a host cell of the present invention expressing the one or more polypeptide of interest is used as a source of the polypeptide(s).

The present invention is further described by the following Examples that should not be construed as limiting the scope of the invention.

EXAMPLES

**Materials and Methods**

General methods of PCR, cloning, ligation, nucleotides etc. are well-known to a person skilled in the art and may for example be found in 'Molecular Cloning: A Laboratory Manual', Sambrook et al. (1988), Cold Spring Harbor Lab., Cold Spring Harbor, NY; Ausubel, F.M. et al. (eds.); 'Current Protocols in Molecular Biology', John Wiley and Sons (1995); Harwood , C.R., and Cutting, S.M. (eds.); 'DNA Cloning: A Practical Approach, Volumes I and II', D.N. Glover ed. (1985); 'Oligonucleotide Synthesis', M.J. Gait ed. (1984); 'Nucleic Acid Hybridization', B.D. Hames & S.J. Higgins eds (1985); 'A Practical Guide To Molecular Cloning', B. Perbal (1984).

Chemicals used as buffers and substrates were commercial products of at least reagent grade.

Aspergillus transformation

Aspergillus transformation was done as described in US 9,487,767. Transformants that had repaired the target niaD-gene and contained the pyrG gene were selected for its ability to grow on minimal plates containing nitrate as nitrogen source and thiamine (Cove D.J., 1966. Biochem. Biophys. Acta 113:51-56). After 5-7 days of growth at 30 degrees C, stable transformants appeared as vigorously growing and sporulating colonies. Transformants were purified through conidiation.

Strain cultivation

The transformed cells are cultivated in a nutrient medium suitable for production of the lipase protein using methods well known in the art. For example, the cells may be cultivated by shake flask cultivation (in which 10 mL YPD medium (2 g/L yeast extract, 2 g/L peptone and 2 % glucose) were inoculated with spores from a transformant and incubated at 30 degrees C for 4 days), and small-scale or large-scale fermentation (including e.g., batch or fed-batch fermentation) in laboratory or industrial fermentors performed in a suitable medium and under conditions allowing the lipase protein to be expressed and recovered. The cultivation takes place in a suitable nutrient medium comprising carbon and nitrogen sources and inorganic salts, using procedures known in the art. Suitable media are available from commercial suppliers or may be prepared according to published compositions. The lipase protein is secreted into the nutrient medium and can be recovered directly here from.

In-Fusion Cloning

In-Fusion Cloning was done using the In-Fusion cloning kit and manuals supplied by Clontech Laboratories, Inc.

Copy number determination by ddPCR

Copy number determination was performed by ddPCR using BioRad QX200™ Droplet Generator and QX200™ Droplet Reader using Biorad QuantaSoft™ version 1.7.4.0917, according to the manufacturer, using oliC as single copy gene.

SDS-PAGE

Protein production was visualized by SDS-PAGE analysis using Criterion™ XT precast gels, 10% Bis-Tris, from BIO-RAD and was run and stained with Coomassie blue as recommended by the manufacturer.

Plasmids

pAT652 is described in Example 1.

pAT1509 is described in Example 1.

Strain

The A. *oryzae* host strain is derived from BECh2 which is described in WO 2000/39322.

**Example 1. Increased production of lipase in *Aspergillus oryzae* using an expression cassette containing two lipase molecules in one mRNA (tandem construct)**

The purpose of this experiment was to construct a plasmid for expression of the lipase lipVR originally isolated from *Valsaria rubricosa* (WO 2018/150021, mature polypeptide of SEQ ID NO: 5) as tandem construct in an *A. oryzae* strain, *i.e.,* containing two lipase molecules in a single polypeptide (Fig. 1). The rationale for this is that if signal peptide removal and translocation of newly synthesized polypeptides into the endoplasmic reticulum (ER) is a rate limiting step for protein secretion in fungi, coupling one signal peptide to two or more polypeptides of interest should aid in increasing secretion.

1A. Construction of pAT652 and pAT1509 for expression of lipVR as singlet or tandem

The purpose of this experiment was to construct a plasmid for expression of lipVR (WO 2018/150021) in an *A. oryzae* strain.

Plasmid pAT652 (Fig. 2) containing the native sequence of lipVR (including its native signal sequence) was constructed by using the In-Fusion Cloning® HD EcoDryTM cloning kit to clone an amplified lipVR gene (SEQ ID NO: 3) using primers lipVR-A (SEQ ID NO: 7) and lipVR-B (SEQ ID NO: 8) into a NotI+AsiSI-digested plasmid derived from pCOls1175 (WO 2013/178674).

Plasmid pAT1509, containing downstream of the Pna2 promoter sequence, the following elements 1) the native lipVR-gene lacking the stop-codon (where the native signal peptide sequence was replaced with the signal peptide sequence of Cutinase pre-pro-sequence (CPP)), followed by a KexB-cleavage sequence (coding for amino acids Lys-Arg), a new DNA sequence coding for the Cutinase Pro sequence devoid of the pre-region and finally followed by a second, codon-optimised, lipVR gene (optimized for A. *oryzae* and lacking introns) (Fig. 3). The lipVR-tandem expression cassette (SEQ ID NO: 9) was constructed by Splicing by Overlap Extension amplification (Higuchi et al, 1988; Horton et al., 2013) using primers lipVR-C (SEQ ID NO: 10) and lipVR-D (SEQ ID NO: 11) into a NotI+AsiSI-digested plasmid derived from pCOls1175 (WO 2013/178674).

### 1B. Construction and analysis of A. *oryzae* strains expressing lipVR as singlet or tandem in multiple copies

Plasmids pAT652 (singlet) and pAT1509 (tandem) were individually used for transformation of A. *oryzae* host strains, and transformants were selected for its ability to grow on sucrose medium supplemented with sodium nitrate (as nitrogen source) and thiamine (down-regulation expression of pyrG-marker for increased copy number preference), but lacking uridine (selection for pyrG complementation).

Selected transformants were cultivated in 10 mL YPD for 4 days at 30 °C and supernatants were analysed for lipase production by SDS-PAGE. Identified transformants producing lipase were genetically characterized for the copy number of the introduced expression construct and selected for fermentation. Strain AT969 contains 18 copies of the singlet lipVR cassette inserted into the genome (or 1 SP per LipVR unit), while strain AT1684 contains 9 copies of the tandem lipVR cassette, thereby 18 LipVR "units" made as 9 tandem molecules with a single SP (or 1 SP per 2 LipVR units). Samples collected from fermentation at day 7 were analyzed for lipase production by SDS-PAGE and lipolytic activity assays (Fig. 4). As shown, a significant increase of secreted LipVR in the tandem strain AT1684 compared to the singlet strain AT969 (75% increased lipolytic activity, Fig. 4).

These results strongly suggest that the use of a tandem lipVR expression cassette dramatically increases the yield of secreted and functionally active enzyme under strong expression conditions at relevant high copy numbers in a production strain background.

### Example 2. Increased enzyme production and conditions for correct processing of the enzyme units

The purpose of this experiment was to investigate whether A. *oryzae* strains like AT1684 constructed using multiple copies of a tandem expression cassette and a suitable promoter such as the Pna2 promoter (WO 2012/160093) can be used not only to increase enzyme yields (see Example 1), but also to ensure correct processing of the given polypeptide of interest, thus enabling the use of this approach within industrial enzyme production where it is important that the produced enzyme molecules are functional and identical in order to ensure uniform activity.

LipVR is a 30 kDa protein that runs at the apparent molecular weight of 40 kDa. Fermentation of AT1684 was performed at two different temperatures (30 and 34 °C) and two different pH values (6.5 and 7.4). MS analysis was performed on the culture supernatants to investigate the composition of LipVR derived molecules. As shown in Fig. 5, a high level of correct processing of LipVR occurs in all conditions tested after 7 days of cultivation. Remarkably, complete processing of LipVR was obtained at pH 6.5 regardless of the growth temperature, providing evidence that these growth conditions are sufficient for the processing of a tandem protein without, e.g., the requirement for overexpression of KexB. The exact conditions (temperature, pH, etc.) required for complete processing by KexB will depend on the fungal host cell of choice. The skilled person will be able to optimize the conditions for any given fungal host cell of choice using methods known in the art.

### Conclusion

When comparing A. *oryzae* strains containing identical copy numbers of the same polypeptide of interest configured as either one signal peptide-one polypeptide of interest (singlet) or one signal peptide-two polypeptide of interest (tandem), a two-fold increase in polypeptide yield was observed with the tandem configuration. Moreover, using nucleic acid constructs of the present invention leads to correct and uniform processing of each copy of the polypeptide of interest at different growth conditions. These results provide unequivocal evidence that remarkable yield increase of industrial functional enzymes can be obtained using genetic constructions containing one SP and more than one CDS of the enzyme gene of interest separated by protease cleavage recognition sites. Additionally, we demonstrate that correct and complete processing of the polypeptide occurs leading to the production of a single and functional LipVR molecule.

### REFERENCES:

Aviram N, Schuldiner M (2017). Targeting and translocation of proteins to the endoplasmic reticulum at a glance.

J Cell Sci 130: 4079-4085.

Higuchi R, Krummel B, Saiki R (1988). A general method of in vitro preparation and specific mutagenesis of DNA fragments: study of protein and DNA interactions. Nucleic Acids Res. 16 (15): 7351-7367.

Horton RM, Cai Z, Ho SN, and Pease LR (2013). Gene Splicing by Overlap Extension: Tailor-Made Genes Using the Polymerase Chain Reaction. BioTechniques 54 (3): 129-133.

Voss M, Schröder B, Fluhrer R (2013). Mechanism, specificity and physiology of signal peptide peptidase (SPP) and SPP-like proteases. Bioch Biophys Acta 1828: 2828-2839.

## Claims

1. A nucleic acid construct comprising a heterologous promoter operably linked to:

   a) a polynucleotide encoding a signal peptide; and
   b) at least two polynucleotides encoding one or more polypeptide of interest;

   wherein the at least two polynucleotides encoding one or more polypeptide of interest are each separated by a linker polynucleotide encoding a linker polypeptide comprising a proteolytic cleavage site;

   wherein the signal peptide, the one or more polypeptide of interest and the linker polypeptide(s) comprising a proteolytic cleavage site are encoded in frame as a single polypeptide, and
   wherein the at least two polynucleotides encode at least two polypeptides of interest consisting of the same amino acid sequence, and wherein the at least two polypeptides of interest are secreted as separate polypeptides consisting of the same amino acid sequence.

2. The nucleic acid construct according to claim 1, wherein the signal peptide has a sequence identity of at least 80% to SEQ ID NO: 2; preferably the signal peptide comprises or consists of SEQ ID NO: 2.

3. The nucleic acid construct according to any of the preceding claims, wherein the polynucleotide encoding the signal peptide has a sequence identity of at least 80% to SEQ ID NO: 1; preferably the polynucleotide encoding the signal peptide comprises or consists of SEQ ID NO: 1.

4. The nucleic acid construct according to any of the preceding claims, wherein the one or more polypeptide of interest comprises an enzyme; preferably the enzyme is selected from the group consisting of hydrolase, isomerase, ligase, lyase, oxidoreductase, or transferase; more preferably an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, and beta-xylosidase; most preferably the one or more polypeptide of interest is a lipase.

5. The nucleic acid construct according to any of the preceding claims, wherein the one or more polypeptide of interest has a sequence identity of at least 80% to the mature polypeptide of SEQ ID NO: 5; preferably the one or more polypeptide of interest comprises or consists of the mature polypeptide of SEQ ID NO: 5.

6. The nucleic acid construct according to any of the preceding claims, wherein the at least two polynucleotides encoding one or more polypeptide of interest have a sequence identity of, independently, at least 80% to the mature polypeptide coding sequence of SEQ ID NO: 4; preferably the at least two polynucleotides encoding one or more polypeptide of interest comprise or consist of the mature polypeptide coding sequence of SEQ ID NO: 4.

7. The nucleic acid construct according to any of the preceding claims, wherein the linker polypeptide comprises at least 10 amino acids.

8. The nucleic acid construct according to any of the preceding claims, wherein the proteolytic cleavage site is a dibasic amino acid motif; preferably the proteolytic cleavage site is a Lys-Arg motif or an Arg-Arg motif.

9. The nucleic acid construct according to any of the preceding claims, wherein the proteolytic cleavage site is a KexB cleavage site.

10. An expression vector comprising a nucleic acid construct according to any of the preceding claims.

11. A fungal host cell comprising a nucleic acid construct according to claims 1-9 and/or an expression vector according to claim 10.

12. The fungal host cell according to claim 11, said fungal host cell being a yeast host cell; preferably the yeast host cell is selected from the group consisting of *Candida, Hansenula, Kluyveromyces, Pichia, Saccharomyces, Schizosaccharomyces,* and *Yarrowia* cell; more preferably the yeast host cell is selected from the group consisting of *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* and *Yarrowia lipolytica* cell.

13. The fungal host cell according to claim 11, said fungal host cell being a filamentous fungal host cell; preferably the filamentous fungal host cell is selected from the group consisting of *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* and *Trichoderma* cell; more preferably the filamentous fungal host cell is selected from the group consisting of *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* and *Trichoderma viride* cell; even more preferably the filamentous host cell is selected from the group consisting of *Aspergillus niger, Aspergillus oryzae, Fusarium venenatum,* and *Trichoderma reesei;* most preferably the filamentous fungal host cell is an *Aspergillus oryzae* cell.

14. A method of producing one or more polypeptide of interest, said method comprising:

   a) providing a fungal host cell according to any of claims 11-13;
   b) cultivating said host cell under conditions conducive for expression of the one or more polypeptide of interest; and optionally
   c) recovering the one or more polypeptide of interest.

15. The method according to claim 14, wherein the one or more polypeptide of interest comprises an enzyme; preferably the enzyme is selected from the group consisting of hydrolase, isomerase, ligase, lyase, oxidoreductase, or transferase; more preferably an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, nuclease, oxidase, pectinolytic enzyme, peroxidase, phosphodiesterase, phytase, polyphenoloxidase, proteolytic enzyme, ribonuclease, transglutaminase, xylanase, and beta-xylosidase; most preferably the one or more polypeptide of interest is a lipase.

## Patentansprüche

1. Nukleinsäurekonstrukt, das einen heterologen Promotor umfasst, der funktionsfähig verknüpft ist mit:

   a) einem Polynukleotid, das ein Signalpeptid kodiert; und
   b) mindestens zwei Polynukleotiden, die ein oder mehrere Polypeptide von Interesse kodieren;

wobei die mindestens zwei Polynukleotide, die ein oder mehrere Polypeptide von Interesse kodieren, jeweils durch ein Linker-Polynukleotid getrennt sind, das ein Linker-Polypeptid kodiert, das eine proteolytische Spaltstelle umfasst;

wobei das Signalpeptid, das eine oder die mehreren Polypeptide von Interesse und das (die) Linker-Polypeptid(e), das (die) eine proteolytische Spaltstelle umfassen, im Leserahmen als ein einziges Polypeptid kodiert sind, und

wobei die mindestens zwei Polynukleotide mindestens zwei Polypeptide von Interesse kodieren, die aus der gleichen Aminosäuresequenz bestehen, und wobei die mindestens zwei Polypeptide von Interesse als separate Polypeptide sezerniert werden, die aus der gleichen Aminosäuresequenz bestehen.

2. Nukleinsäurekonstrukt nach Anspruch 1, wobei das Signalpeptid eine Sequenzidentität von mindestens 80% zu SEQ ID NO: 2 aufweist; bevorzugt umfasst das Signalpeptid SEQ ID NO: 2 oder besteht daraus.

3. Nukleinsäurekonstrukt nach einem beliebigen der voranstehenden Ansprüche, wobei das Polynukleotid, das das Signalpeptid kodiert, eine Sequenzidentität von mindestens 80% zu SEQ ID NO: 1 aufweist; bevorzugt umfasst das Polynukleotid, das das Signalpeptid kodiert, SEQ ID NO: 1 oder besteht daraus.

4. Nukleinsäurekonstrukt nach einem beliebigen der voranstehenden Ansprüche, wobei das eine oder die mehreren Polypeptide von Interesse ein Enzym umfassen; bevorzugt ist das Enzym ausgewählt aus der Gruppe bestehend aus Hydrolase, Isomerase, Ligase, Lyase, Oxidoreduktase oder Transferase; stärker bevorzugt eine Aminopeptidase, Amylase, Carbohydrase, Carboxypeptidase, Katalase, Cellobiohydrolase, Cellulase, Chitinase, Cutinase, Cyclodextrin-Glycosyltransferase, Desoxyribonuclease, Endoglucanase, Esterase, alpha-Galactosidase, beta-Galactosidase, Glucoamylase, alpha-Glucosidase, beta-Glucosidase, Invertase, Laccase, Lipase, Mannosidase, Mutanase, Nuclease, Oxidase, pektinolytisches Enzym, Peroxidase, Phosphodiesterase, Phytase, Polyphenoloxidase, proteolytisches Enzym, Ribonuclease, Transglutaminase, Xylanase und beta-Xylosidase; am stärksten bevorzugt ist das eine oder mehrere Polypeptide von Interesse eine Lipase.

5. Nukleinsäurekonstrukt nach einem beliebigen der voranstehenden Ansprüche, wobei das eine oder die mehreren Polypeptide von Interesse eine Sequenzidentität von mindestens 80% zum reifen Polypeptid von SEQ ID NO: 5 aufweist; bevorzugt umfasst das eine oder die mehreren Polypeptide von Interesse das reife Polypeptid von SEQ ID NO: 5 oder besteht daraus.

6. Nukleinsäurekonstrukt nach einem beliebigen der voranstehenden Ansprüche, wobei die mindestens zwei Polynukleotide, die ein oder mehrere Polypeptide von Interesse kodieren, unabhängig voneinander eine Sequenzidentität von mindestens 80% zur das reife Polypeptid kodierenden Sequenz von SEQ ID NO: 4 aufweisen; bevorzugt umfassen die mindestens zwei Polynukleotide, die ein oder mehrere Polypeptide von Interesse kodieren, die das reife Polypeptid kodierende Sequenz von SEQ ID NO: 4 oder bestehen daraus.

7. Nukleinsäurekonstrukt nach einem beliebigen der voranstehenden Ansprüche, wobei das Linker-Polypeptid mindestens 10 Aminosäuren umfasst.

8. Nukleinsäurekonstrukt nach einem beliebigen der voranstehenden Ansprüche, wobei die proteolytische Spaltstelle ein dibasisches Aminosäuremotiv ist; bevorzugt ist die proteolytische Spaltstelle ein Lys-Arg-Motiv oder ein Arg-Arg-Motiv.

9. Nukleinsäurekonstrukt nach einem beliebigen der voranstehenden Ansprüche, wobei die proteolytische Spaltstelle eine KexB-Spaltstelle ist.

10. Expressionsvektor, der ein Nukleinsäurekonstrukt gemäß einem beliebigen der voranstehenden Ansprüche umfasst.

11. Pilzwirtszelle, die ein Nukleinsäurekonstrukt gemäß Ansprüchen 1-9 und/oder einen Expressionsvektor gemäß Anspruch 10 umfasst.

12. Pilzwirtszelle nach Anspruch 11, wobei die Pilzwirtszelle eine Hefewirtszelle ist; bevorzugt ist die Hefewirtszelle ausgewählt aus der Gruppe bestehend aus einer *Candida-, Hansenula-, Kluyveromyces-, Pichia-, Saccharomyces-, Schizosaccharomyces-* und Yarrowia-Zelle; stärker bevorzugt ist die Hefewirtszelle ausgewählt aus der Gruppe, bestehend aus einer *Kluyveromyces lactis-, Saccharomyces carlsbergensis-, Saccharomyces cerevisiae-, Saccharomyces diastaticus-, Saccharomyces douglasii-, Saccharomyces kluyveri-, Saccharomyces norbensis-, Saccha-*

*romyces oviformis-* und *Yarrowia lipolytica-Zelle.*

13. Pilzwirtszelle nach Anspruch 11, wobei die Pilzwirtszelle eine Wirtszelle eines filamentösen Pilzes ist; bevorzugt ist die Wirtszelle eines filamentösen Pilzes ausgewählt aus der Gruppe bestehend aus einer *Acremonium-, Aspergillus-, Aureobasidium-, Bjerkandera-, Ceriporiopsis-, Chrysosporium-, Coprinus-, Coriolus-, Cryptococcus-, Filibasidium-, Fusarium-, Humicola-, Magnaporthe-, Mucor-, Myceliophthora-, Neocallimastix-, Neurospora-, Paecilomyces-, Penicillium-, Phanerochaete-, Phlebia-, Piromyces-, Pleurotus-, Schizophyllum-, Talaromyces-, Thermoascus-, Thielavia-, Tolypocladium-, Trametes-,* und *Trichoderma-*Zelle; stärker bevorzugt ist die Wirtszelle eines filamentösen Pilzes ausgewählt aus der Gruppe bestehend aus einer *Aspergillus awamori-, Aspergillus foetidus-, Aspergillus fumigatus-, Aspergillus japonicus-, Aspergillus nidulans-, Aspergillus niger-, Aspergillus oryzae-, Bjerkandera adusta-, Ceriporiopsis aneirina-, Ceriporiopsis caregiea-, Ceriporiopsis gilvescens-, Ceriporiopsis pannocinta-, Ceriporiopsis rivulosa-, Ceriporiopsis subrufa-, Ceriporiopsis subvermispora-, Chrysosporium inops-, Chrysosporium keratinophilum-, Chrysosporium lucknowense-, Chrysosporium merdarium-, Chrysosporium pannicola-, Chrysosporium queenslandicum-, Chrysosporium tropicum-, Chrysosporium zonatum-, Coprinus cinereus-, Coriolus hirsutus-, Fusarium bactridioides-, Fusarium cerealis-, Fusarium crookwellense-, Fusarium culmorum-, Fusarium graminearum-, Fusarium graminum-, Fusarium heterosporum-, Fusarium negundi-, Fusarium oxysporum-, Fusarium reticulatum-, Fusarium roseum-, Fusarium sambucinum-, Fusarium sarcochroum-, Fusarium sporotrichioides-, Fusarium sulphureum-, Fusarium torulosum-, Fusarium trichothecioides-, Fusarium venenatum-, Humicola insolens-, Humicola lanuginosa-, Mucor miehei-, Myceliophthora thermophila-, Neurospora crassa-, Penicillium purpurogenum-, Phanerochaete chrysosporium-, Phlebia radiata-, Pleurotus eryngii-, Thielavia terrestris-, Trametes villosa-, Trametes versicolor-, Trichoderma harzianum-, Trichoderma koningii-, Trichoderma longibrachiatum-, Trichoderma reesei-,* und Trichoderma viride-Zelle; noch stärker bevorzugt ist die Wirtszelle eines filamentösen Pilzes ausgewählt aus der Gruppe bestehend aus *Aspergillus niger, Aspergillus oryzae, Fusarium venenatum* und *Trichoderma reesei;* am stärksten bevorzugt ist die Wirtszelle eines filamentösen Pilzes eine *Aspergillus* oryzae-Zelle.

14. Verfahren zum Herstellen eines oder mehrerer Polypeptide von Interesse, wobei das Verfahren umfasst

    a) Bereitstellen einer Pilzwirtszelle gemäß einem beliebigen der Ansprüche 11-13;
    b) Kultivieren der Wirtszelle unter Bedingungen, die für die Expression des einen oder der mehreren Polypeptide von Interesse förderlich sind; und gegebenenfalls
    c) Gewinnen des einen oder der mehreren Polypeptide von Interesse.

15. Verfahren nach Anspruch 14, wobei das eine oder die mehreren Polypeptide von Interesse ein Enzym umfassen; bevorzugt ist das Enzym ausgewählt aus der Gruppe bestehend aus Hydrolase, Isomerase, Ligase, Lyase, Oxidoreduktase oder Transferase; stärker bevorzugt eine Aminopeptidase, Amylase, Carbohydrase, Carboxypeptidase, Katalase, Cellobiohydrolase, Cellulase, Chitinase, Cutinase, Cyclodextrin-Glycosyltransferase, Desoxyribonuclease, Endoglucanase, Esterase, alpha-Galactosidase, beta-Galactosidase, Glucoamylase, alpha-Glucosidase, beta-Glucosidase, Invertase, Laccase, Lipase, Mannosidase, Mutanase, Nuclease, Oxidase, pektinolytisches Enzym, Peroxidase, Phosphodiesterase, Phytase, Polyphenoloxidase, proteolytisches Enzym, Ribonuclease, Transglutaminase, Xylanase und beta-Xylosidase; am stärksten bevorzugt ist das eine oder die mehreren Polypeptide von Interesse eine Lipase.

## Revendications

1. Produit d'assemblage d'acide nucléique comprenant un promoteur hétérologue lié de manière fonctionnelle à :

    a) un polynucléotide codant un peptide signal ; et
    b) au moins deux polynucléotides codant un ou plusieurs polypeptides d'intérêt ;

dans lequel les au moins deux polynucléotides codant un ou plusieurs polypeptides d'intérêt sont séparés par un polynucléotide lieur codant un polypeptide lieur comprenant un site de coupure protéolytique ;

    dans lequel le peptide signal, le ou les polypeptides d'intérêt et le ou les polypeptides lieurs comprenant un site de coupure protéolytique sont codés dans le cadre sous la forme d'un seul polypeptide, et
    dans lequel les au moins deux polynucléotides codent au moins deux polypeptides d'intérêt consistant en la même séquence d'acides aminés, et dans lequel les au moins deux polypeptides d'intérêt sont sécrétés sous la forme de polypeptides séparés consistant en la même séquence d'acides aminés.

**2.** Produit d'assemblage d'acide nucléique selon la revendication 1, dans lequel le peptide signal a une identité de séquence d'au moins 80 % avec la SEQ ID NO : 2, de préférence le peptide signal comprend ou consiste en la SEQ ID NO : 2.

**3.** Produit d'assemblage d'acide nucléique selon l'une quelconque des revendications précédentes, dans lequel le polynucléotide codant le peptide signal a une identité de séquence d'au moins 80 % avec la SEQ ID NO : 1 ; de préférence le polynucléotide codant le peptide signal comprend ou consiste en la SEQ ID NO : 1.

**4.** Produit d'assemblage d'acide nucléique selon l'une quelconque des revendications précédentes, dans lequel le ou les polypeptides d'intérêt comprend une enzyme ; de préférence l'enzyme est choisie dans le groupe constitué par une hydrolase, une isomérase, une ligase, une lyase, une oxydoréductase, ou une transférase ; de préférence une aminopeptidase, une amylase, une carbohydrase, une carboxypeptidase, une catalase, une cellobiohydrolase, une cellulase, une chitinase, une cutinase, une cyclodextrine glycosyltransférase, une désoxyribonucléase, une endoglucanase, une estérase, une alpha-galactosidase, une bêta-galactosidase, une glucoamylase, une alpha-glucosidase, une bêta-glucosidase, une invertase, une laccase, une lipase, une mannosidase, une mutanase, une nucléase, une oxydase, une enzyme pectinolytique, une peroxydase, une phosphodiestérase, une phytase, une polyphénol oxydase, une enzyme protéolytique, une ribonucléase, une transglutaminase, une xylanase, et une bêta-xylosidase ; plus préférentiellement le ou les polypeptides d'intérêt est une lipase.

**5.** Produit d'assemblage d'acide nucléique selon l'une quelconque des revendications précédentes, dans lequel le ou les polypeptides d'intérêt ont une identité de séquence d'au moins 80 % avec le polypeptide mature de la SEQ ID NO : 5 ; de préférence le ou les polypeptides d'intérêt comprennent ou consistent en le polypeptide mature de la SEQ ID NO : 5.

**6.** Produit d'assemblage d'acide nucléique selon l'une quelconque des revendications précédentes, dans lequel les au moins deux polynucléotides codant un ou plusieurs polypeptides d'intérêt ont une identité de séquence, indépendamment, d'au moins 80 % avec la séquence codante du polypeptide mature de la SEQ ID NO : 4 ; de préférence les au moins deux polynucléotides codant un ou plusieurs polypeptides d'intérêt comprennent ou consistent en la séquence codante du polypeptide mature de la SEQ ID NO : 4.

**7.** Produit d'assemblage d'acide nucléique selon l'une quelconque des revendications précédentes, dans lequel le polypeptide lieur comprend au moins 10 acides aminés.

**8.** Produit d'assemblage d'acide nucléique selon l'une quelconque des revendications précédentes, dans lequel le site de coupure protéolytique est un motif d'acides aminés dibasiques ; de préférence le site de coupure protéolytique est un motif Lys-Arg ou un motif Arg-Arg.

**9.** Produit d'assemblage d'acide nucléique selon l'une quelconque des revendications précédentes, dans lequel le site de coupure protéolytique est un site de coupure KexB.

**10.** Vecteur d'expression comprenant un produit d'assemblage d'acide nucléique selon l'une quelconque des revendications précédentes.

**11.** Cellule hôte fongique comprenant un produit d'assemblage d'acide nucléique selon les revendications 1 à 9 et/ou un vecteur d'expression selon la revendication 10.

**12.** Cellule hôte fongique selon la revendication 11, ladite cellule hôte fongique étant une cellule hôte de levure ; de préférence la cellule hôte de levure est choisie dans le groupe constitué par les cellules de *Candida, Hansenula, Kluyveromyces*, *Pichia, Saccharomyces, Schizosaccharomyces,* et *Yarrowia* ; plus préférentiellement la cellule hôte de levure est choisie dans le groupe constitué par les cellules de *Kluyveromyces lactis, Saccharomyces carlsbergensis, Saccharomyces cerevisiae, Saccharomyces diastaticus, Saccharomyces douglasii, Saccharomyces kluyveri*, *Saccharomyces norbensis, Saccharomyces oviformis,* et *Yarrowia lipolytica.*

**13.** Cellule hôte fongique selon la revendication 11, ladite cellule hôte fongique étant une cellule hôte de champignon filamenteux ; de préférence la cellule hôte de champignon filamenteux est choisie dans le groupe constitué par les cellules *d'Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora*, *Paecilomyces, Penicillium, Phanerochaete, Phlebia, Piromyces, Pleurotus, Schizophyllum*, *Talaromyces,*

*Thermoascus, Thielavia, Tolypocladium, Trametes*, et *Trichoderma* ; plus préférentiellement la cellule hôte de champignon filamenteux est choisie dans le groupe constitué par les cellules d'*Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* et *Trichoderma viride* ; plus préférentiellement encore la cellule hôte de champignon filamenteux est choisie dans le groupe constitué par *Aspergillus niger, Aspergillus oryzae, Fusarium venenatum,* et *Trichoderma reesei* ; plus préférentiellement la cellule hôte de champignon filamenteux est une cellule d'*Aspergillus oryzae.*

14. Méthode de production d'un ou plusieurs polypeptides d'intérêt, ladite méthode comprenant :

   a) l'obtention d'une cellule hôte fongique selon l'une quelconque des revendications 11 à 13 ;
   b) la culture de ladite cellule hôte dans des conditions propices à l'expression du ou des polypeptides d'intérêt ; et éventuellement
   c) la récupération du ou des polypeptides d'intérêt.

15. Méthode selon la revendication 14, dans laquelle le ou les polypeptides d'intérêt comprend une enzyme ; de préférence l'enzyme est choisie dans le groupe constitué par une hydrolase, une isomérase, une ligase, une lyase, une oxydoréductase, et une transférase ; de préférence une aminopeptidase, une amylase, une carbohydrase, une carboxypeptidase, une catalase, une cellobiohydrolase, une cellulase, une chitinase, une cutinase, une cyclodextrine glycosyltransférase, une désoxyribonucléase, une endoglucanase, une estérase, une alpha-galactosidase, une bêta-galactosidase, une glucoamylase, une alpha-glucosidase, une bêta-glucosidase, une invertase, une laccase, une lipase, une mannosidase, une mutanase, une nucléase, une oxydase, une enzyme pectinolytique, une peroxydase, une phosphodiestérase, une phytase, une polyphénol oxydase, une enzyme protéolytique, une ribonucléase, une transglutaminase, une xylanase, et une bêta-xylosidase ; plus préférentiellement le ou les polypeptides d'intérêt est une lipase.

Fig. 1

Fig. 2

promoter (thiA-Ao)

promoter NA2

5'-UTR (thiA-Ao)

5'-UTR(tpiA-Ao)

pyrG (AO)

lipVR-tandem

terminator (pyrG-Ao)

bla-promoter (pUC19)

pAT1509

terminator (amg-An)

LEU2 (Sc)

terminator (niaD-Ao)

Origin

Delta niaD (86-868aa)

Fig. 3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018150021 A
- WO 9600787 A
- WO 0056900 A
- US 6011147 A
- WO 9533836 A
- WO 2010039889 A
- WO 0024883 A
- EP 238023 A
- US 9487767 B
- WO 200039322 A
- WO 2013178674 A
- WO 2012160093 A

### Non-patent literature cited in the description

- **RABOUILLE.** *Trends in Cell Biology,* 2017, vol. 27, 230-240
- **KIM et al.** *Journal of Cell Science,* 2018, vol. 131
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **ROMANOS et al.** *Yeast,* 1992, vol. 8, 423-488
- **GUO ; SHERMAN.** *Mol. Cellular Biol.,* 1995, vol. 15, 5983-5990
- **INNIS et al.** PCR: A Guide to Methods and Application. Academic Press, 1990
- **GEMS et al.** *Gene,* 1991, vol. 98, 61-67
- **CULLEN et al.** *Nucleic Acids Res.,* 1987, vol. 15, 9163-9175
- **HAWKSWORTH et al.** Ainsworth and Bisby's Dictionary of The Fungi. CAB International, University Press, 1995
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- **BECKER ; GUARENTE.** Guide to Yeast Genetics and Molecular Biology, Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989
- **SAMBROOK et al.** Molecular Cloning: A Laboratory Manual. Cold Spring Harbor Lab, 1988
- Current Protocols in Molecular Biology. John Wiley and Sons, 1995
- DNA Cloning: A Practical Approach. 1985
- Oligonucleotide Synthesis. 1984
- Nucleic Acid Hybridization. 1985
- **B. PERBAL.** A Practical Guide To Molecular Cloning. 1984
- **COVE D.J.** *Biochem. Biophys. Acta,* 1966, vol. 113, 51-56
- **AVIRAM N ; SCHULDINER M.** Targeting and translocation of proteins to the endoplasmic reticulum at a glance. *J Cell Sci,* 2017, vol. 130, 4079-4085
- **HIGUCHI R ; KRUMMEL B ; SAIKI R.** A general method of in vitro preparation and specific mutagenesis of DNA fragments: study of protein and DNA interactions. *Nucleic Acids Res.,* 1988, vol. 16 (15), 7351-7367
- **HORTON RM ; CAI Z ; HO SN ; PEASE LR.** Gene Splicing by Overlap Extension: Tailor-Made Genes Using the Polymerase Chain Reaction. *BioTechniques,* 2013, vol. 54 (3), 129-133
- **VOSS M ; SCHRÖDER B ; FLUHRER R.** Mechanism, specificity and physiology of signal peptide peptidase (SPP) and SPP-like proteases. *Bioch Biophys Acta,* 2013, vol. 1828, 2828-2839